# EUROPEAN PATENT SPECIFICATION

(11) **EP 2 501 696 B1**
(45) Date of publication and mention of the grant of the patent: **28.12.2016**
(21) Application number: 10771403.2
(22) Date of filing: 15.10.2010
(51) Int. Cl.: C07D 405/04, C07D 409/04, C07D 413/04, C07D 417/04, A61K 51/04

(54) **IMAGING AGENTS AND THEIR USE FOR THE DIAGNOSTIC IN VIVO OF NEURODEGENERATIVE DISEASES, NOTABLY ALZHEIMER'S DISEASE AND DERIVATIVE DISEASES**
KONTRASTMITTEL UND IHRE VERWENDUNG ZUR IN-VIVO-DIAGNOSE VON NEURODEGENERATIVEN ERKRANKUNGEN, IM BESONDEREN MORBUS ALZHEIMER UND DARAUS RESULTIERENDE ERKRANKUNGEN
AGENTS POUR L'IMAGERIE ET LEUR UTILISATION POUR LE DIAGNOSTIC IN VIVO DE MALADIES NEURODÉGÉNÉRATRICES, NOTAMMENT LA MALADIE D'ALZHEIMER ET DES MALADIES APPARENTÉES

(30) Priority: 15.10.2009 US 251916 P
(43) Date of publication of application: 26.09.2012
(73) Proprietor: Guerbet, 93420 Villepinte (FR)
(72) Inventor: CATOEN, Sarah, F-93190 Livry Gargan (FR); GAUTHERET, Thierry, F-77590 Bois Le Roi (FR)
(74) Representative: Lavoix
(86) International application number: PCT/EP2010/065526
(87) International publication number: WO 2011/045415

(56) References cited:
- EP-A1- 2 274 983
- WO-A1-01/00610
- WO-A1-03/004023
- WO-A1-2005/105798
- WO-A1-2006/066172
- WO-A1-2008/000643
- WO-A1-2009/042092
- WO-A1-2009/061856
- WO-A1-2010/125985
- WO-A2-2007/033080
- DE-A1- 10 050 663
- DE-A1- 19 920 936
- GB-A- 2 311 010
- US-A1- 2010 261 727
- YONA, RODRIGUE LEUMA ET AL: "Thioflavin derivatives as markers for amyloid -.beta. fibrils: insights into structural features important for high-affinity binding", CHEMMEDCHEM , 3(1), 63-66 CODEN: CHEMGX; ISSN: 1860-7179, vol. 3, no. 1, 2008, pages 63-66, XP002632939,
- ORITA K ET AL: "Synthesis and evaluation of 1-{1-[5-(2'-[<18>F]Fluoroethyl)-2-thienyl ]-cyclohexyl}piperidine as a potential in vivo radioligand for the NMDA receptor-Channel complex", NUCLEAR MEDICINE AND BIOLOGY, ELSEVIER, NY, US, vol. 20, no. 7, 1 October 1993 (1993-10-01), pages 865-873, XP023258443, ISSN: 0969-8051, DOI: DOI:10.1016/0969-8051(93)90153-L [retrieved on 1993-10-01]
- SEGGIO A ET AL: "Palladium-catalyzed cross-couplings of lithium arylzincates with aromatic halides: Synthesis of analogues of isomeridianin G and evaluation as GSK-3[beta] inhibitors", SYNTHESIS 2009 GEORG THIEME VERLAG DEU LNKD- DOI:10.1055/S-0029-1217003, no. 21, 8 September 2009 (2009-09-08), pages 3617-3632, XP002632940, ISSN: 0039-7881
- MACDONALD D ET AL: "Hunting the Emesis and Efficacy Targets of PDE4 Inhibitors: Identification of the Photoaffinity Probe 9-(3-azidophenyl)-6-[(4-iod o-1H-1-imidazolyl)methyl]quinoline (APIIMQ)", JOURNAL OF MEDICINAL CHEMISTRY, AMERICAN CHEMICAL SOCIETY, WASHINGTON, US, vol. 43, 30 September 2000 (2000-09-30), pages 3820-3823, XP002173935, ISSN: 0022-2623, DOI: DOI:10.1021/JM000065C
- HISANO TAKUZO ET AL: "Synthesis of benzoaxazoles, benzothiazoles and benzimidazoles and evaluation of their antifungal, insecticidal and herbicidal activities", CHEMICAL AND PHARMACEUTICAL BULLETIN, PHARMACEUTICAL SOCIETY OF JAPAN, JP, vol. 30, 1 January 1982 (1982-01-01), pages 2996-3004, XP002170422, ISSN: 0009-2363

## Description

The present invention relates to new imaging agents and their use for the *in vivo* diagnostic of neurodegenerative diseases, notably Alzheimer's disease and related diseases.

Alzheimer's disease (AD) is the most common form of dementia in elderly. Nowadays, several millions of patients suffer from AD and this number is expected to increase exponentially with the lengthening mean life span. AD is a slow progressive neurodegenerative brain disorder characterized by irreversible memory loss, deterioration of cognitive function along with behavioural symptoms, language impairment and disorientation.

Post-mortem examination of AD brain sections reveals abundant extracellular senile plaques (SPs) and numerous intraneuronal neurofibrillary tangles (NFTs) ; both of them along with activated microglia and reactive astrocytes have been commonly accepted as the hallmark of AD [1,2].

SPs originate from insoluble neurotoxic deposits of Ab40 and Ab42 peptides on neurons ('Ab-plaque' or 'amyloid plaque'), resulting from cleavage of the amyloid precursor protein (APP) by specific proteases whereas NFTs are formed by filaments of highly phosphorylated tau proteins.

Clinical diagnosis of AD based on neurological observations by neuropsychological tests such as Mini-Mental State Examination is often difficult, unreliable and only yields indirect information. Since the deposition of Ab plaques is an early event in the development of AD, a validated biomarker of Ab deposition in the brain would be very helpful for identifying and following individuals at risk for AD and for assisting the evaluation of new anti-amyloid therapies currently under development. Therefore, detection and quantitative evaluation of Ab plaques in the brain with non-invasive techniques such as positron emission tomography (PET) and single photon emission computed tomography (SPECT) is useful for presymptomatic identification of patients and monitoring the effectiveness of novel treatments.

To allow non-invasive *in vivo* diagnosis of AD, radiolabelled derivatives of a number of immunohistochemical dyes (Congo red, chrysamine G, thioflavin S, or thioflavin T) have been tested and reported [4]. X-34 derivatives consisting of only half the X-34 molecule without carboxylic groups, the so-called stilbenes (SBs), showed promising results [8]. The reported carbon-11 labelled SB-13 has already been tested in subjects with mild to moderate AD and healthy controls, showing a high accumulation in the frontal and temporoparietal cortex of patients with AD but not in age-matched control subjects [9]. Barrio and co-workers, on the other hand, developed [18F]FDDNP, a naphtalene derivative that binds to amyloid at a different binding site and also to the NFTs [10]. It was reported that non-ionic analogues of Thioflavin T, an ionic imaging dye, penetrate the blood-brain barrier (BBB) and show high affinity for Ab-plaque. The most promising of all reported compounds seems to be the carbon-11 labelled 6-OH-BTA or 6-hydroxy-2-(40-N-[11C]methylaminophenyl)-1,3-benzothiazole, also known as Pittsburgh Compound-B (PIB). PIB has already been tested intensively in several clinical studies showing clear differences between AD, mild cognitive impairment (MCI) and control subjects [12]. However, this agent is labelled with short-lived carbon-11 (t1/2 = 20.39 min), which limits its availability to centres equipped with a cyclotron. This limitation may be overcome by introducing a fluorine-18 label which has a longer half-life (t1/2 = 109.8 min) and thus allows to provide a positron emission tomography (PET) tracer that is useful for a widespread clinical application: early clinical evaluation of fluorine-18 labelled derivatives (BAY94-9172, Av-45, GE-067) is also ongoing [13-15].

Many of the known amyloid targeting compounds are polycyclic compounds that contain a first part A consisting in a 6 or 5 membered cycle fused with or substituted by an other 6 or 5 membered cycle, linked to a second part B that is typically an aromatic mono or polycyclic group, leading to compounds (I) illustrated in the following formula :

(A)-(B).

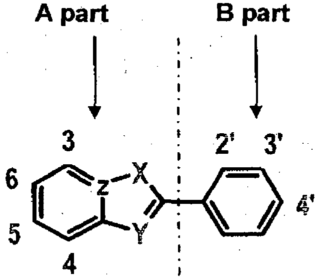

Known scaffolds comprising heteroatoms N, O or S at the X,Y, Z positions are notably benzothiazoles, benzofuranes, benzothiophenes, aminopyridines also described notably in patent documents WO2007/033080, WO 2007/124345, WO2007/047204, WO2008/134618, WO2008/118122 WO 2007/086800, WO 2008/0657875, WO 2007/011834, WO 2003/068269, WO 2008/124812, WO 2008/073350, WO 2007/045593, WO 2007/126733.

Known compounds of the prior art are for instance the following.

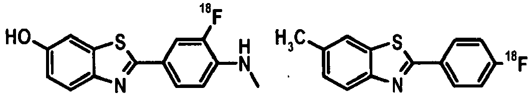

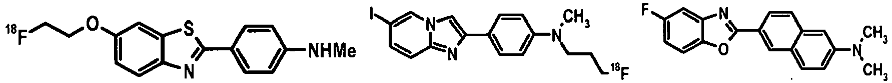

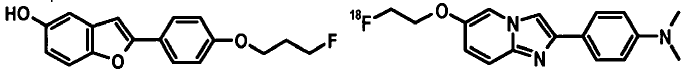

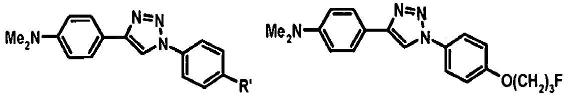

According to the knowledge of the applicant, when the aromatic cycle B of the prior art is a 6 membered cycle, the atom of the cycle B at the position 4' is a C atom which is substituted by a Y group that is frequently a group NR1 R2 (R1 and R2 being notably a H atom or an alkyl group). The Y groups are described in the literature as stabilizing groups and/or as groups that are useful for the biological targeting affinity. Recent documents WO 2007/086800 or WO2007/126733 describe some 6 membered B heterocycles that contain N atoms replacing some of the C atoms. However, the N atoms are described in positions 2', 3', 5', 6', but not at the 4' position. Further, B groups disclosed in the prior art are consistently chosen from 6 membered ring structures.

Yona et al. (ChemMedChem 2008, 3, 63), Orita et al. (nuclear medecine and biology, 20(7), 1993, 865), Seggio et al. (Synthesis, 2009, 21, 3617) and applications WO 2009/042092, WO 2005/105798, WO 03/004023, DE 100 50 663, WO 01/00610, WO 2008/000643, DE 199 20 936, US 2010/261727 and WO 2007/033080 describe compounds for dignosing and/or treating Alzheimer's disease.

Yona et al. describe a compound having the following formula:

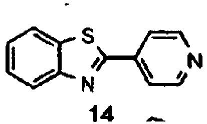

Orita et al. describe radioligands for the NMDA receptor-channel complex having a role in Alzheimer's disease, and in particular a compound having the following formula:

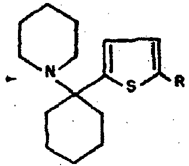

(10) R = CH₂CH₂¹⁸F (¹⁸FE-TCP)

Seggio et al. describe Glycogen Synthase Kinase 3 (GSK-3β) inhibitors involved, in Alzheimer's disease. The following compounds are described:

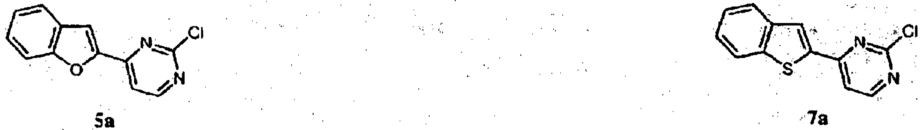

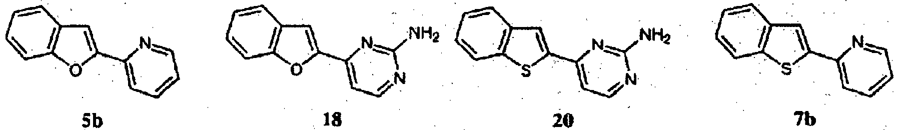

Some compounds exemplified in application WO 2009/042092 comprise an indole cycle linked to a 4-pyridinyle group.

The following compounds are described in the examples of application WO 2005/105798: 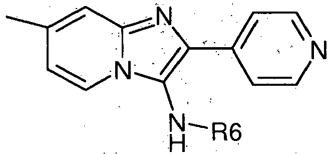 R6 = heptyl (expl 24)
octyl (expl 25)
1,1-diméthylhexyl (expl 26)

The following compounds are described in the examples of application WO 03/004023:

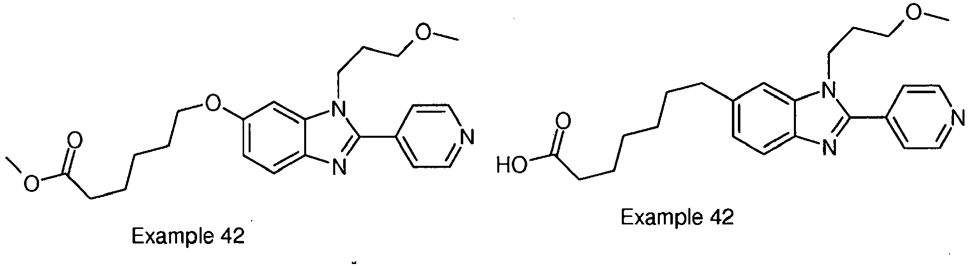

Some compounds exemplified in application DE 100 50 663 comprise an imidazo[1,2-a]pyridine cycle linked to a 4-pyridinyle group.

Some compounds exemplified in applications WO 01/00610, WO 2008/000643 and DE 199 20 936 comprise a benzimidazole cycle linked to a 4-pyridinyle group.

Some compounds exemplified in application US 2010/261727 comprise a benzothiophene cycle linked to a 4-pyridinyle group.

Application WO 2007/033080 describes compounds for imaging amyloid deposits having the following formula :

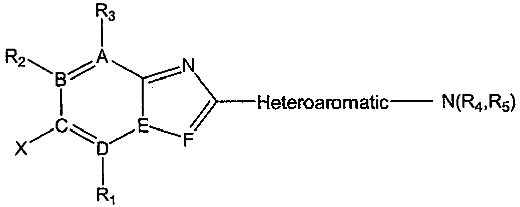

Application GB 2322010 describes the compound 4-(5-trifluorom6thylbenzothiazol-2-yl)pyridine useful as a precursor in the synthesis of compounds for diagnosing and treating Alzheimer's disease.

Application WO 2009/061856 describes non-nucleoside reverse transcriptase inhibitors (NNRTI) for treating HIV infection. Some exemplified compounds comprise an imidazopyridine cycle linked to a 4-pyridinyle group.

Application WO 2006/066172 describes compounds for treating cancer. The following compounds are described in the examples:

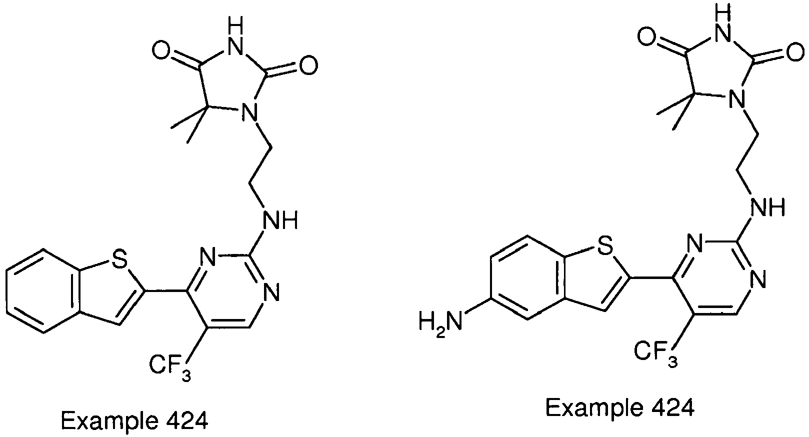

Macdonald et al. (J. Med. Chem. 2000, 43, 3820) describe phosphodiesterase cAMP-specific (PDE4) inhibitors for treating asthma having the following formulae:

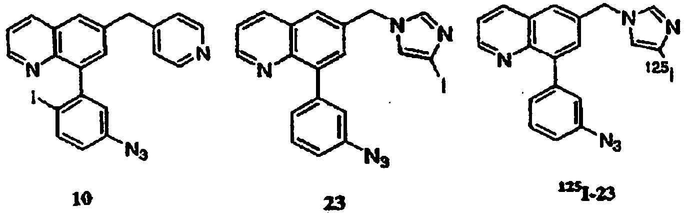

Application EP 2 274 983 and WO 2010/125985 describe compounds useful for controlling harmful arthropod having the following formulae: 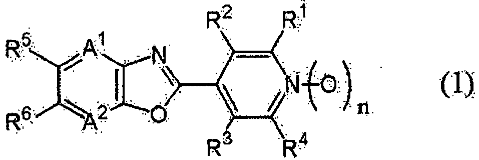 or

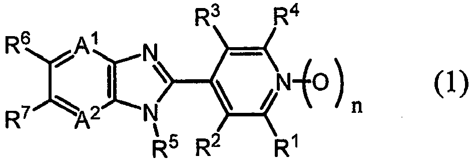

Hisano et al. (Chemical and Pharmaceutical Bulletin, Pharmaceutical Society of Japan, 30, 1982, 2996) describe compounds presenting antifungal, insecticidal and herbicidal activities and of formula: 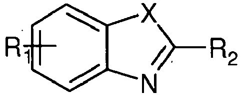 wherein X represents S, R₂ represents 4-pyridyle and R₁ represents 5-CF₃ (example 12) or 7-CF₃ (example 16).Despite the high number of compounds investigated in the prior art, a few of which being at the clinical human stage, there is still a need for novel and efficient compounds, in particular for the early diagnostic of Alzheimer's disease. Among the huge number of possibilities of compounds derivating from those known in the art, the applicant has now worked on specific selected new compounds useful for Alzheimer's disease imaging which have a new B cycle, and lead to promising biological efficiency.

According to a first aspect the invention concerns a compound of formula 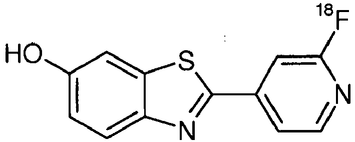 (I);
and its pharmaceutically acceptable salts.

According to another object, the present invention also concerns the process of preparation of the compound of the invention.

The compound and process of the present invention may be prepared in a number of ways well-known to those skilled in the art. The compound can be synthesized, for example, by application or adaptation of the methods described below, or variations thereon as appreciated by the skilled artisan. The appropriate modifications and substitutions will be readily apparent and well known or readily obtainable from the scientific literature to those skilled in the art.

In particular, such methods can be found in R.C. Larock, Comprehensive Organic Transformations, Wiley-VCH Publishers, 1999.

The compound (I) of the invention may be prepared by application or adaptation of the following routes, merely given for illustrative purposes.

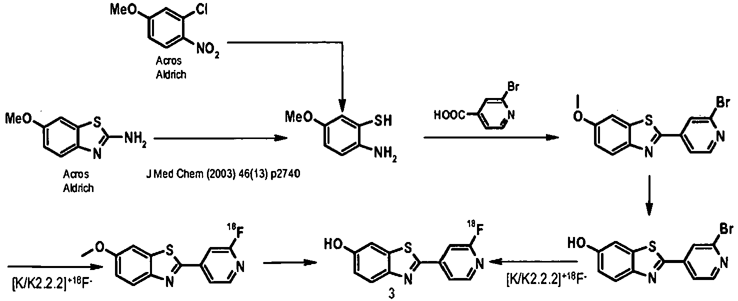

Additionally, the process of the invention may lead to several regioisomers which are all encompassed by the present invention. Regioisomers are generally isolated by chromatography.

The compound of the present invention may be prepared by a variety of synthetic routes. The reagents and starting materials are commercially available, or readily synthesized by well-known techniques by one of ordinary skill in the arts. All substituents, unless otherwise indicated, are as previously defined.

In the reactions described hereinafter, it may be necessary to protect reactive functional groups, for example hydroxyl, amino, imino, thio or carboxy groups, where these are desired in the final product, to avoid their unwanted participation in the reactions. Conventional protecting groups may be used in accordance with standard practice, for examples see T.W. Greene and P. G. M. Wuts in Protective Groups in Organic Chemistry, 3rd ed., John Wiley and Sons, 1999; J. F. W. McOmie in Protective Groups in Organic Chemistry, Plenum Press, 1973.

Some reactions may be carried out in the presence of a base. There is no particular restriction on the nature of the base to be used in this reaction, and any base conventionally used in reactions of this type may equally be used here, provided that it has no adverse effect on other parts of the molecule. Examples of suitable bases include: sodium hydroxide, potassium carbonate, triethylamine, alkali metal hydrides, such as sodium hydride and potassium hydride; alkyllithium compounds, such as methyllithium and butyllithium; and alkali metal alkoxides, such as sodium methoxide and sodium ethoxide.

Usually, reactions are carried out in a suitable solvent. A variety of solvents may be used, provided that it has no adverse effect on the reaction or on the reagents involved. Examples of suitable solvents include: hydrocarbons, which may be aromatic, aliphatic or cycloaliphatic hydrocarbons, such as hexane, cyclohexane, benzene, toluene and xylene; amides, such as dimethylformamide; alcohols such as ethanol and methanol and ethers, such as diethyl ether and tetrahydrofuran.

The reactions can take place over a wide range of temperatures. In general, it is found convenient to carry out the reaction at a temperature of from 0°C to 150°C (more preferably from about room temperature to 100°C). The time required for the reaction may also vary widely, depending on many factors, notably the reaction temperature and the nature of the reagents. However, provided that the reaction is effected under the preferred conditions outlined above, a period of from 3 hours to 20 hours will usually suffice.

The compound thus prepared may be recovered from the reaction mixture by conventional means. For example, the compound may be recovered by distilling off the solvent from the reaction mixture or, if necessary, after distilling off the solvent from the reaction mixture, pouring the residue into water followed by extraction with a water-immiscible organic solvent and distilling off the solvent from the extract. Additionally, the product can, if desired, be further purified by various well-known techniques, such as recrystallization, reprecipitation or the various chromatography techniques, notably column chromatography or preparative thin layer chromatography.

The radionuclide reagent may be chosen from any reagents generally used for this purpose and known from the skilled person, in particular K18F/K222, Rb18F, Cs18F, R4N+18F; more particularly, the F18 reagent is the so called K18F/K222, commercially available from Merck (Kriptofix ®).

The reaction is typically a nucleophilic substitution.

The reaction may generally be carried out in appropriate solvents such as acetonitrile, DMSO, DMF, sulfolan, dimethylacetamide. The conditions are advantageously : heating 80-180°C for less then 30min, or microwave activation (100W, 1-2 min).

This reaction is generally conducted quickly, as the half life of F18 is 119.8 minutes.

In situ, the radiopharmacist couples these compounds with the radionuclide produced typically by a cyclotron (for instance radioactive ¹⁸F), to make the final compound labelled with the radionuclide and then ready for administration to the patient.

### Definitions

As used herein, Hal or halo, respectively fluoro, iodo, bromo, chloro represent halogen, respectively F, I, Br, Cl, including all isotopes thereof.

As used herein, "pharmaceutically acceptable" is employed to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, "pharmaceutically acceptable salts" refer to derivatives of the disclosed compound wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, phosphoric, and the like; and the salts prepared from organic acids such as lactic, maleic, citric, benzoic, methanesulfonic, and the like.

The pharmaceutically acceptable salts of the present invention can be synthesized from the parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are used.

As used herein "stable compound" and "stable structure" are meant to indicate a compound that is sufficiently robust to survive isolation to a useful degree of purity from a reaction mixture, and subsequent prolonged storage in the cold or at ambient temperature, and optionally formulated into an efficacious therapeutic or diagnostic agent.

Examples of an "effective amount" include amounts that enable imaging of amyloid deposit(s) *in vivo,* that yield acceptable toxicity and bioavailability levels for pharmaceutical use, and/or prevent cell degeneration and toxicity associated with fibril formation.

Further details of method of use of the compound of the applicant (doses, protocols of administration ...) are also know by the one skilled in the art, reminded notably in WO2008/108729.

The compound of the present invention may be used to determine the presence, location and/or amount of one or more amyloid deposit(s) in an organ or body area, including the brain, of an animal or human. Amyloid deposit(s) include, without limitation, deposit(s) of A[β]. In allowing the temporal sequence of amyloid deposition to be followed, the compound of the invention may also be used to correlate amyloid deposition with the onset of clinical symptoms associated with a disease, disorder or condition. The inventive compound may be used to prevent, treat and/or to diagnose a disease, disorder or condition characterized by amyloid deposition, such as AD. The compounds of formula (I) has several potential targets including NFTs and SPs, relating to Ab plaques and/or Tau aggregates, useful notably for the early diagnostic of AD.

The compound for use of the invention determines the presence and location of amyloid deposits in an organ or body area, preferably brain, of a patient. The present compound for use comprises administration of a detectable quantity of a pharmaceutical composition containing an amyloid-binding compound of the present invention called a "detectable compound," or a pharmaceutically acceptable water-soluble salt thereof, to a patient. A "detectable quantity" means that the amount of the detectable compound that is administered is sufficient to enable detection of binding of the compound to amyloid. An "imaging effective quantity" means that the amount of the detectable compound that is administered is sufficient to enable imaging of binding of the compound to amyloid.

The invention employs amyloid probes which, in conjunction with non-invasive neuroimaging techniques such as gamma imaging such as positron emission tomography (PET) or single-photon emission computed tomography (SPECT), and eventually MRI, are used to quantify amyloid deposition *in vivo.*

In addition to a high affinity for amyloid b plaques, the preferred tracer agents of the invention may exhibit several features :
- they are preferably able to cross the blood-brain barrier (BBB). This requires advantageously a neutral molecule with a molecular mass not exceeding 600 ;
- the log octanol-buffer partition coefficient, which is a measure of the lipophilicity of the compound, should be preferably between 1 and 3.

The radiolabelled compound of the invention can be detected using gamma imaging wherein emitted gamma irradiation of the appropriate wavelength is detected. Methods of gamma imaging include, but are not limited to, SPECT and PET.

The compound of the present invention may be administered by any means known to one of ordinary skill in the art. For example, administration to the subject may be local or systemic and accomplished orally, parenterally, by inhalation spray, topically, rectally, nasally, buccally, vaginally, or via an implanted reservoir. The term "parenteral" as used herein includes subcutaneous, intravenous, intraarterial, intramuscular, intraperitoneal, intrathecal, intraventricular, intrasternal, intracranial, and intraosseous injection and infusion techniques. The exact administration protocol will vary depending upon various factors including the age, body weight, general health, sex and diet of the patient; the determination of specific administration procedures would be routine to any one of ordinary skill in the art.

According to a further object, the present invention further provides a pharmaceutical composition, in particular for *in vivo* imaging of amyloid deposits, comprising a labelled compound (I) (in an effective amount) together with a pharmaceutically acceptable carrier.

The composition may comprise one or more additional pharmaceutically acceptable ingredient(s), including without limitation one or more wetting agent(s), buffering agent(s), suspending agent(s), lubricating agent(s), emulsifier(s), disintegrant(s), absorbent(s), preservative(s), surfactant(s), colorant(s), flavorant(s), sweetener(s) and therapeutic agent(s). In one embodiment, the composition is formulated for intravenous administration and the carrier includes a fluid and/or a nutrient replenisher.

In another embodiment, the composition is capable of binding specifically to amyloid *in vivo,* is capable of crossing the blood-brain barrier, is non-toxic at appropriate dose levels and/or has a satisfactory duration of effect.

In yet another embodiment, the composition comprises about 10 mg of human serum albumin and from about 0.0005 to 500 mg of a compound (I) of the present invention per mL of phosphate buffer containing NaCl.

Dose levels on the order of about 0.001 µg/kg/day to about 10,000 mg/kg/day of an inventive compound are useful for the inventive methods. In one embodiment, the dose level is about 0.001 µg/kg/day to about 10 g/kg/day. In another embodiment, the dose level is about 0.01 µg/kg/day to about 1.0 g/kg/day. In yet another embodiment, the dose level is about 0.1 mg/kg/day to about 100 mg/kg/day.

Any known administration regimen for regulating the timing and sequence of drug delivery may be used and repeated as necessary to effect treatment in the inventive methods. The regimen may include pretreatment and/or co-administration with additional therapeutic agent(s).

In one embodiment, the compound (I) is administered to a subject that is suspected of having or that is at risk of developing a disease, disorder or condition characterized by amyloid deposition. Typically, the subject may be an elderly human.

The present invention further provides compounds for use diagnosing, treating or preventing an Aβ-related pathology in a patient, comprising administering to the patient a therapeutically effective amount of a labelled compound of formula (I).

The present invention further provides a labelled compound of formula (I) described herein for diagnosing, treating or preventing an Aβ-related pathology. The present invention further provides a compound of formula (I) described herein for the manufacture of a medicament, in particular a diagnostic contrast agent.

The present invention further provides :
- a compound for use in the treatment or preventing an A[β]-related pathology in a patient, comprising administering to the patient a therapeutically effective amount of a labelled compound of formula (I) ;
- the use of a pharmaceutical composition of the invention for *in vivo* imaging carried out by the group of techniques selected from gamma imaging, magnetic resonance imaging and magnetic resonance spectroscopy, preferably PET imaging ;
- the use of a labelled compound (I) in the manufacture of a medicament for diagnostic, prevention and/or treatment of Alzheimer's disease ;
- a labelled compound (I) for use in determining the efficacy of therapy in the treatment of Alzheimer's disease ;
- a labelled compound (I) described herein for use as a medicament.

In the present application, the terms "A[β]-related pathology" or "Alzheimer's disease" or "amyloidosis" refers in particular to Alzheimer's disease and known related diseases which comprise Downs syndrome, a [β]-amyloid angiopathy, cerebral amyloid angiopathy, hereditary cerebral hemorrhage, a disorder associated with cognitive impairment, MCI ("mild cognitive impairment"), Alzheimer Disease, memory loss, attention deficit symptoms associated with Alzheimer disease, neurodegeneration associated with Alzheimer disease, dementia of mixed vascular origin, dementia of degenerative origin, pre-senile dementia, senile dementia, dementia associated with Parkinson's disease, progressive supranuclear palsy or cortical basal degeneration.

In still another embodiment, there is provided a compound for use in identifying a patient as prodromal to a disease associated with amyloid deposition comprising:
(A) administering to the patient, who is presenting with signs of clinical dementia or clinical signs of a mild cognitive impairment, an amyloid binding labelled compound of formula (I) or a pharmaceutically acceptable salt thereof; then
(B) imaging said patient to obtain data; and
(C) analyzing said data to ascertain amyloid levels in said patient with reference to a normative level, thereby identifying said patient as prodromal to a disease associated with amyloid deposition.

In still another embodiment, alone or in combination with any other embodiment herein described, the invention provides the use of a labelled compound according to formula (I), as herein defined, for determining the efficacy of therapy in the treatment of amyloidosis. Another embodiment is the use of a formula (I) labelled compound in the preparation of a medicament for determining the efficacy of therapy in the treatment of amyloidosis. The method comprises typically :
(A) administering to a patient in need thereof an effective amount of an amyloid binding labelled compound of formula (I) or a pharmaceutically acceptable salt thereof;
(B) imaging said patient; then
(C) administering to said patient in need thereof at least one anti-amyloid agent;
(D) subsequently administering to said patient in need thereof an effective amount of a labelled compound of formula (I);
(E) imaging said patient; and
(F) comparing levels of amyloid deposition in said patient before treatment with said at least one anti-amyloid agent to levels of amyloid deposition in said patient after treatment with said at least one anti-amyloid agent.

The present invention relates to the compound of formula (I) as hereinbefore defined as well as to the salts thereof. Salts for use in pharmaceutical compositions will be pharmaceutically acceptable salts, but other salts may be useful in the production of the compound of formula (I).

The compounds of the invention can be used as medicaments. In some embodiments, the present invention provides the compound of formula (I), or pharmaceutically acceptable salts, tautomers thereof, for use as medicaments.

Further, the labelled compound (I) may be administered in combination, at the same time or at a differed time with other imaging or therapeutic agents targeting Alzheimer's disease. For instance they can be used before or after a MRI contrast agent, notably a nanoparticle such as an iron oxyde nanoparticle (eventually coated with biovectors such as peptides and/or with PEG groups or aminoalcool groups) that are able to target amyloid plaques or inflammatory zones associated to amyloid plaques. For instance the MRI agent is administered first and the PET imaging agent (I) is injected afterwards or the other way round. The compound and eventually other diagnostic agents may be administered in different areas of the brain presumed to be linked to the same or to a different stage of the disease. Any appropriate mapping of the disease may be advantageously constructed. Different known imaging modalities for Alzheimer's disease such as cerebral blood volume methodologies may be also used.

It is reminded that for the radiolabelling, the one skilled in the art is also aware of techniques described notably in the following documents and references quoted in the present application :
- Klunk, W.E. and Mathis, C.A.Jr (2007) Preparation of isotopically labelled benzothiazole compounds as imaging agents for amyloidogenic proteins.
- Mason, N.S., et al. (2007) Synthesis and evaluation of [18F]-PIB analogs as A beta plaque PET imaging agents. J Label Compd Radiopharm. 50(Suppl1), S87.
- Stephenson, K.A. et al. (2007) Fluoro-pegylated (FPEG) imaging agents targeting Abeta aggregates. Bioconjug Chem. 18(1), 238-46.
- Cai, L. et al. (2004) Synthesis and evaluation of two 18F-labelled 6-iodo-2-(4'-N,N-dimethylamino)phenylimidazo[1,2-a]pyridine derivatives as prospective radioligands for beta-amyloid in Alzheimer's disease. J Med Chem. 47(9), 2208-18.

The following example of synthesis of the compound is given for illustrative, nonlimiting purposes.

### 1) Chemical synthesis

The compound (I) of the invention may be prepared according to the following routes :

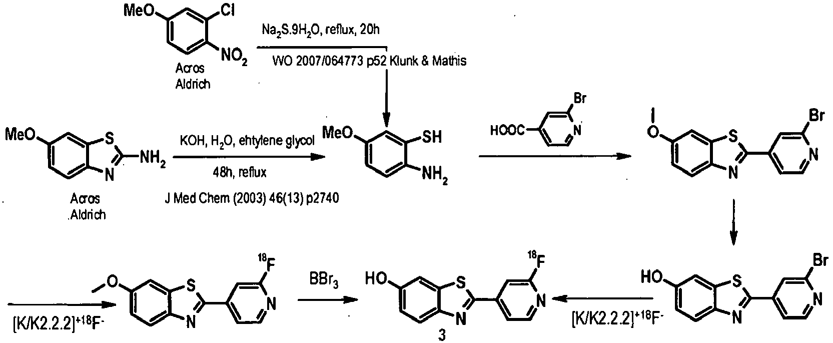

Referring to the general description the labelled compound (I) of formula 3 obtained is :

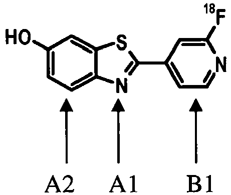

It may be obtained according to the following protocol :
**2-Amino-5-methoxythiophenol.** 2-Amino-6-methoxy-benzothiazole (10 g, 57 mmol) was suspended in 50% KOH (60 g KOH dissolved in 60 mL water) and ethylene glycol (15mL). The suspension was heated to reflux for 48 h. Upon cooling to room temperature, toluene (100 mL) was added and the reaction mixture was neutralized with acetic acid (60 mL). The organic layer was separated, and the aqueous layer was extracted with toluene. The toluene layers were combined and washed with water and dried over MgSO₄. Evaporation of the solvent gave 5 g of 2-amino-5-methoxythiophenol as yellow solid. 1 H NMR (300 MHz, DMSO-d6) δ: 6.72 (d, 1 H), 6.54 (d, 1 H), 6.37 (dd, 1 H), 3.85 (s, 3 H) ; MS (ES) m/z (M+H) 156.4
**2-(2-Bromopyridin-4-yi)-6-methoxy-1,3-benzothiazole.** 2-bromo-4-pyridinecarboxylic acid (1.9 g, 12.7 mmol) and 2-amino-5-methoxythiophenol (2 g, 13.3 mmol) were mixed together with PPA (5g) and heated to 170 °C under N₂ atmosphere for 2h. The reaction mixture was cooled to room temperature and poured into 10% K₂CO₃ solution. The precipitate was filtered under reduced pressure. The crude product was purified by flash column chromatography (0 to 2% methanol in DCM) to give the 2-(2-Bromopyridin-4-yl)-6-methoxy-1,3-benzothiazole (1.1 g). 1H NMR (300 MHz, DMSO-d6) δ: 8.92 (d, 1H) 8.63 (d, 1 H) 7.95 (d, 1 H) 7.76 (d, 1 H) 7.42 (dd, 1 H) 7.11 (dd, 1 H) 3.89 (s, 3H) ; MS (ES) *m*/*z* (M+H) 322.
**2-(2-Bromopyridin-4-yl)-6-hydroxy-1,3-benzothiazole** To a solution of 2-(2-Bromopyridin-4-yl)-6-methoxy-1,3-benzothiazole (500 mg) in DCM (anhydrous, 10 mL) was added BBr₃ (20 mL, 1.0 M DCM solution) was injected at 0°C under N₂ atmosphere. The reaction mixture was allowed to warm slowly and stirred at room temperature overnight. After water was added, the reaction mixture was stirred for another 0.5 h. The organic layer was separated, and the aqueous layer was extracted with DCM. The combined organic layers were dried over MgSO₄, evaporated *in vacuo,* and the crude product was purified by prep. HPLC to give 2-(2-Bromopyridin-4-yl)-6-hydroxy-1,3-benzothiazole (250 mg). 1 H NMR (300 MHz, DMSO) δ: 9.65 (s, 1 H) 8.95 (d, 1 H) 8.65 (d, 1 H) 7.83 (d, 1 H) 7.51 (d, 1 H) 7.31 (dd, 1 H) 7.18 (dd, 1 H) ; MS (ES) *m*/*z* (M+H) 308.2.
**2-(2-[¹⁸F]Fluoropyridin-4-yl)-6-hydroxy-1,3-benzothiazole.** [18F]Fluoride, produced by a cyclotron using 18O(p,n)18F reaction, was passed through a Sep-Pak Light QMA cartridge as an aqueous solution in [180]-enriched water. The cartridge was dried by airflow, and the 18F activity was eluted with 2 ml of Kryptofix 222 (K222)/K2CO3 solution (28 mg of K222 and 2.5 mg of K2CO3 in 0.75 ml CH3CN/H2O 5:95 v/v). The solvent was removed at 120°C for 7 min under an argon stream. The residue was azeotropically dried with 1 ml of anhydrous CH3CN twice at 120°C under an argon stream.

A solution of bromo precursor (4 mg) in DMF (0.5 ml) was added to the reaction vessel containing the dried 18F activities. The solution was heated at 100°C for 15 min in a closed vial to provide the crude radiolabeled compound.

The mixture was cooled down to room temperature and after dilution with an equal volume of 0.05 M ammonium acetate purified with RP-HPLC using an XTerra Prep RP18 10 mm x250 mm column (Waters) eluted isocratically with a mixture of 50% 0.05 M NH4OAc and 50% ethanol/tetrahydrofuran (75:25 v/v) at a flow rate of 3 mL/min. The fraction containing the isolated radioactive compound was diluted with an equal volume of water and then applied on an activated Sep-Pak_ Plus C18 cartridge (Waters) that was rinsed with 10 ml water and then eluted with 1 ml ethanol. The purity of the labeled tracers was analyzed using an XTerra RP18 5 µm, 4.6 mm x 250 mm column (Waters) eluted with an isocratic mixture of 50% 0.05 M NH4OAc and 50% ethanol/tetrahydrofuran (75:25 v/v) at a flow rate of 1 mL/min (Rt [18F]Cpd = 28.5 min)

The preparation took 90 min and the radiochemical yield was 20% (decay corrected). The average specific activity was found to be 105 GBq/µmol at end of synthesis.

### 2) Biological activity

The following protocol is used for testing *in vitro* binding of the compound by using Aβ (1-42) aggregated peptides in solution.

### 2.1 Preparation of peptides Aβ (1-42)

The solid form of peptide Aβ (1-42) is gently dissolved at 50 µM in a sterile buffer solution (pH 7.4) containing 10 mM sodium phosphate and 1 mM EDTA. Small aliquots (200 µL) are frozen and stored at -20°C until their use. An aliquot of peptide is thawed and incubated for 36-42h at 37°C under gentle and constant shaking. This incubation leads to aggregation of peptides. At the end of the incubation time, aggregated peptides are diluted in the buffer solution in order to obtain the concentrations of 20 µM. The addition of 50 µL of this solution (final volume of 1 mL) allows obtaining the testing concentrations of 1 µM.

### 2.2 Preparation of the non radioactive ligands

A solution of test compounds is prepared at 3 mM in DMSO. These solutions are diluted in ethanol 10% in order to obtain the testing concentrations of 0.3, 1, 3, 10, 30, 100, 300 and 1000 nM.

### 2.3 Preparation of the radioactive ligand (IMPY)

The ¹²⁵I-radiolabelled ligand solution is diluted in ethanol 10% in order to obtain the final concentration of 0.05 nM in IMPY.

At the beginning of the experiment, 1 µL of ¹²⁵I-radiolabelled IMPY solution will be added to 199 µL of ethanol 10% and is counted with a gamma-counter. Fifty microliters of the working solution of ¹²⁵I-radiolabelled IMPY is added to 150 µL of ethanol 10% and is counted (background). The results obtained are compared to the theorical values and the working solution is adjusted by adding radiolabelled IMPY or ethanol 10% if needed. A variation of 10% will be accepted between theorical and experimental values.

### 2.4 Incubation of aggregated peptides with test compounds and ¹²⁵I-radiolabelled IMPY.

Fifty microliters of aggregated peptides (20 µM) are incubated for 3h under gentle agitation at room temperature with 40 µL of each non-radiolabelled test compound dilution and with 50 µL of radiolabelled ligand at 1 nM in 860 µL of ethanol 10% (quadruplicate *per* condition). For the total binding, 50 µL of aggregated peptides (20 µM) is incubated for 3h under gentle agitation at room temperature with 40 µL of ethanol 10% and with 50 µL of radiolabelled ligand solution at 1 nM in 860 µL of ethanol 10% (quadruplicate *per* condition). Tubes (quadruplicate) without aggregated peptides are added as control of radioactivity retained on filter. Fifty microliters of peptides buffer solution are mixed with 40 µL ethanol 10% and with 50 µL of the radiolabelled ligand solution in 860 µL of ethanol 10% (quadruplicate *per* condition).

At the end of incubation time, the mixture is then filtered through GF/B filters by using a Brandel M24 Harvester. Filters are then washed twice with 3 mL of ethanol 10%. Filters are collected and the radioactivity is counted with a gamma-counter Cobra II.

A curve is drawn with GraphPad Prism 4.02 software in order to represent the total specific radioligand binding (cpm) as a function of the logarithm concentration of unlabelled compound. This curve allows determining the IC₅₀ for each compound. The IC₅₀ is the concentration of unlabelled test compound that blocks 50% of the specific binding. The Ki of IMPY (homologous competition) is calculated from the IC₅₀, using the following equation: Kᵢ = K_{D} = IC₅₀ - L. The Ki of each compound is calculated from the IC₅₀, using the following equation: Ki = IC₅₀ / [1 + [radioligand]/ K_{D}].

### 2.5 Compounds testing

Positive results and binding values (range of 1 to 5 on a [0-5] scale) indicate promising affinity towards amyloid markers (or other AD associated markers such as Tau aggregates), with values IC50 in the target range 1 to 300 nM, in particular 5 to 100 nM. Values Log P of about 2 to 4 may be obtained.

### 2.6 Comparaison of compound of formula (I) and the PIB compound

The most examined compound PIB having the following formula : 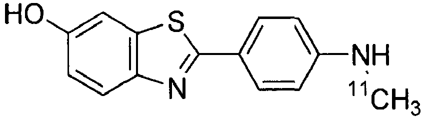 has been tested clinically and, hence demonstrated to be a potential biomarker for the visualization of Aβ plaques in AD brains with PET. However, the first generation of radioligands for PET, including the [¹¹C]PIB, are not ideal for quantification due to low signal to noise ratio, high non-specific binding or unfavourable kinetics. The non-specific binding of Aβ PET ligands should also be minimized in order to increase the sensitivity to allow for even lower levels of Aβ plaques to be detected, and thus to monitor β-amyloid lowing therapies with higher sensitivity.

Therefore, a critical need to fine-tune the binding specificity, the kinetics of the uptake and washout of Aβ radioligands derivatives exists. Previous results regarding uptake into and clearance from the brain point to high lipophilicity as one of the reasons for a slow washout from the brain and a high non-specific binding, two crucial properties for achieving an adequate signal to noise ratio.

The present inventors replaced the original phenyl group in phenyl derivatives with a less lipophilic pyridyl group (compounds of formula (I)) to reduce non-specific binding and increased wash-out rate of non-specific binding *in vivo.*

It is well known that electron-withdrawing substituents, such as a nitro or carbonyl group, present on the ortho or para position of a nitrophenyl ring, activate the nitro group and facilitate the exchange of the nitro group for a fluorine-18 atom : radiolabelling of a 4'-nitrophenyl (cycle B) derivative via a direct aromatic nucleophilic substitution with fluorine-18 would be possible as fused cycle A part could act as electron-withdrawing group. However in some cases, the radiolabeling failed because in the alkaline labelling conditions a partial negative charge is induced at position 6 (cases of amino or hydroxy substituents). As a result, the fused cycle A part no longer acts as electron-withdrawing, substituent : introduction of Boc or MOM protecting groups at position 6 would allow for radiolabelling but implies an additional step of cleavage after the radiolabelling.

To overcome this problem, nucleophilic heteroaromatic substitution at the *ortho-*position (3') with no-carrier-added [18F]fluoride appears as the most efficient method. Like for the aliphatic nucleophilic radiofluorinations, only a good leaving group is required (a halogen, or better a nitro- or a trimethylammonium group). There is no need for an additional strong electron-withdrawing substituent for activation of the aromatic ring such as in the *homo*aromatic nucleophilic radiofluorinations, except if one considers *meta-*fluorination. The compound of formula (I) can therefore be easily synthesized.

Ultimately, introduction of the nitrogen in the 4', position of the (B) cycle allows for keeping ability to form hydrogen bonds with Aβ as with the classical 4'-mono- or dimethylamino amino group: it is generally preferred that the molecules have a hydrogen bond donor both on the left-hand and right-hand side in order to specifically bind to amyloid plaques in AD brain tissue.

Additionally, the presence of the 4' nitrogen instead of the mono- or dimethylamino group in PIB blocks a potential metabolic position where conversion to demethylated metabolites able to cross the blood-brain-barrier might complicate quantification of PET brain images.

### List of main References

1. Hardy, J. A.; Higgins, G. A. Science 1992, 256, 184.
2. Selkoe, D. J. Physiol. Rev. 2001, 81, 741.
4. Mathis, C. A.; Wang, Y.; Klunk, W. E. Curr. Pharm. Des. 2004, 10, 1469.
8. Ono, M.; Wilson, A.; & al Nucl. Med. Biol. 2003, 30, 565.
9. Verhoeff, N.; Wilson, A.; & al Am. J. Geriatr. Psychiatr. 2004, 12, 584.
10. Agdeppa, E.; Kepe, V.; & al J. Neurosci. 2001, 21, 1.
12. Klunk, W. E.; Engler, H.; & al Ann. Neurol. 2004, 55, 306.
13. Rowe, C.; Ackerman, U.; & al Lancet Neurol. 2008, 7, 129.
14. Zhang, W.; Kung, M.P. Nucl. Med. Biol. 2007, 34, 89.
15. Mathis, C.; Lopresti, B.; & al J. Nucl. Med. 2007, 48, 56P.

## Claims

1. Compound of formula 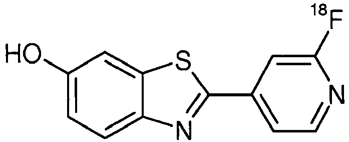 and its pharmaceutically acceptable salts.

2. A pharmaceutical composition comprising a compound according to claim 1 together with a pharmaceutically acceptable carrier.

3. A pharmaceutical composition for *in vivo* imaging of amyloid deposits, comprising a compound according to claim 1, together with a pharmaceutically acceptable carrier.

4. Compound according to claim 1 for use for in vivo imaging by a technique selected from gamma imaging, magnetic resonance imaging and magnetic resonance spectroscopy, preferably PET imaging.

5. Compound according to claim 1 for use for diagnosing, preventing and/or treatment of Alzheimer's disease.

6. Compound according to claim 1 for use in determining the efficacy of therapy in the treatment of Alzheimer's disease.

## Patentansprüche

1. Verbindung der Formel 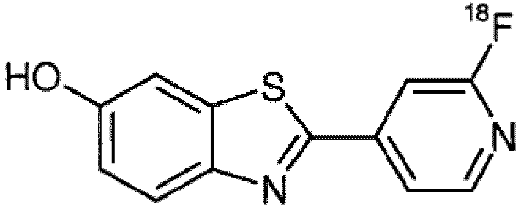 und ihre pharmazeutisch unbedenklichen Salze.

2. Pharmazeutische Zusammensetzung, umfassend eine Verbindung nach Anspruch 1 zusammen mit einem pharmazeutisch unbedenklichen Träger.

3. Pharmazeutische Zusammensetzung zur In-vivo-Bilddarstellung von Amyloidablagerungen, umfassend eine Verbindung nach Anspruch 1 zusammen mit einem pharmazeutisch unbedenklichen Träger.

4. Verbindung nach Anspruch 1 zur Verwendung bei der In-vivo-Bilddarstellung durch ein Verfahren ausgewählt aus Gamma-Bilddarstellung, Magnetresonanz-Bilddarstellung und Magnetresonanz-Spektroskopie, vorzugsweise PET-Bilddarstellung.

5. Verfahren nach Anspruch 1 zur Verwendung bei der Diagnose, Prävention und/oder Behandlung von Alzheimer-Krankheit.

6. Verbindung nach Anspruch 1 zur Verwendung bei der Bestimmung der Wirksamkeit einer Therapie bei der Behandlung von Alzheimer-Krankheit.

## Revendications

1. Composé de formule 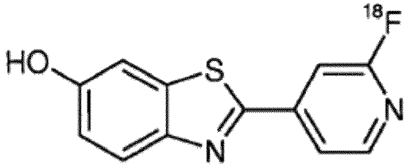 et ses sels pharmaceutiquement acceptables.

2. Composition pharmaceutique comprenant un composé selon la revendication 1 conjointement avec un véhicule pharmaceutiquement acceptable.

3. Composition pharmaceutique pour l'imagerie *in vivo* de dépôts amyloïdes, comprenant un composé selon la revendication 1, conjointement avec un véhicule pharmaceutiquement acceptable.

4. Composé selon la revendication 1 pour utilisation dans l'imagerie *in vivo* par une technique choisie parmi l'imagerie gamma, l'imagerie par résonance magnétique et la spectrométrie par résonance magnétique, de préférence l'imagerie TEP.

5. Composé selon la revendication 1 pour utilisation dans le diagnostic, la prévention et/ou le traitement de la maladie d'Alzheimer.

6. Composé selon la revendication 1 pour utilisation dans la détermination de l'efficacité de la thérapie dans le traitement de la maladie d'Alzheimer.
